# EUROPEAN PATENT SPECIFICATION

(11) **EP 4 154 801 B1**
(45) Date of publication and mention of the grant of the patent: **05.03.2025**
(21) Application number: 22195583.4
(22) Date of filing: 14.09.2022
(51) Int. Cl.: A61B 5/00, A61B 5/11

(54) **SENSOR DEVICE**
SENSORVORRICHTUNG
DISPOSITIF DE CAPTEUR

(30) Priority: 24.09.2021 JP 2021155046
(43) Date of publication of application: 29.03.2023
(73) Proprietor: Casio Computer Co., Ltd., Tokyo 151-8543 (JP)
(72) Inventor: Matsui, Satoshi, Hamura-shi, Tokyo (JP)
(74) Representative: Grünecker Patent- und Rechtsanwälte PartG mbB

(56) References cited:
- JP-A- 2017 015 486
- JP-A- 2020 099 413
- US-A1- 2019 365 285
- US-A1- 2020 060 546

## Description

### BACKGROUND OF THE INVENTION

### Field of the Invention

The present disclosure relates to a sensor device.

### Description of the Related Art

US 2019/365 285 A1 discloses a sensor device comprising a first main body portion and a second main body portion which are configured to be detachably attached to a holding member worn by a user, wherein the first main body portion is disposed on a side of the holding member which lies opposite to a side facing a body of the user and includes therein an antenna for receiving or transmitting a radio wave, and
wherein the second main body portion is disposed on the side of the holding member which lies to face the body of the user, and includes therein a measuring unit configured to measure information of the user.
US 2020 060 546 A1 discloses a sensor device in a clip form having a measuring unit configured to measure information of the user. Further, conventionally, there have been provided sensor devices which are configured to be attached to a holding member (an edge portion of training pants, a belt, or the like) to measure and analyze a movement form or a movement pace of a moving person. For example, a sensor device described in Japanese Patent Laid-Open No. 2020-99413 is made up of an acceleration sensor with a clip provided thereon and is designed to be mounted on a back side of a human body by making use of a waist belt. This sensor device can wirelessly communicate with a terminal device such as a smartphone or the like using a predetermined system.

This sensor device is mounted as close to the human body as possible so as to detect a motion of the human body in a more secured fashion. As a result, the conventional sensor device is mounted on an inner side of the holding member which is a side facing a certain part of the human body so that a casing portion, in which a sensor element is provided, can contact closely the human body. This may result in a case in which an antenna for communication with the terminal device is positioned inside clothing. Then, in the case that the clothing becomes wet due to sweat exuded during exercise or rain, the casing including the antenna is positioned between the human body which is wet due to exuded sweat and the wet clothing. This causes the human body which is wet due to the exuded sweat and the wet clothing to constitute a dielectric, whereby the antenna is disposed between the dielectrics. The transmission and reception status of a radio communication differs between when the clothing is wet and when the clothing is dry. To deal with this problem, when the sensitivity of the antenna is controlled so that the antenna exhibits its optimum performance in an intermediate status between when the clothing is wet and when the clothing is dry, there is caused a problem in that the performance (sensitivity) of the antenna is reduced when the clothing is wet and dry.

### SUMMARY OF THE INVENTION

Above problem is overcome by a sensor device having the features of claim 1. According to an aspect of the present disclosure, there is provided electronic equipment comprising:
a display unit configured to display information using an indicator; and
at least one processor;
wherein the processor is configured to
obtain a number of times of vital pulsation during a predetermined period of time, and
cause the display unit to represent a pulsation period based on the number of times of vital pulsation so obtained using the indicator.

Further features of the present disclosure will become apparent from the following description of exemplary embodiments (with reference to accompanying drawings).

### BRIEF DESCRIPTION OF THE DRAWINGS

FIG. 1A is a perspective view of a sensor device according to an embodiment of the present disclosure as seen from a first main body portion side;
FIG. 1B is a perspective view of the sensor device according to the embodiment of the present disclosure as seen from a side thereof where a lock component is provided;
FIG. 2 is a diagram showing a state in which the sensor device according to the embodiment of the present disclosure is worn by a user;
FIG. 3 is a control block diagram of the sensor device according to the embodiment of the present disclosure; and
FIG.4 is a sectional view of the sensor device according to the embodiment of the present disclosure taken along a line IV-IV in FIG. 2.

### DETAILED DESCRIPTION OF THE PREFERRED EMBODIMENTS

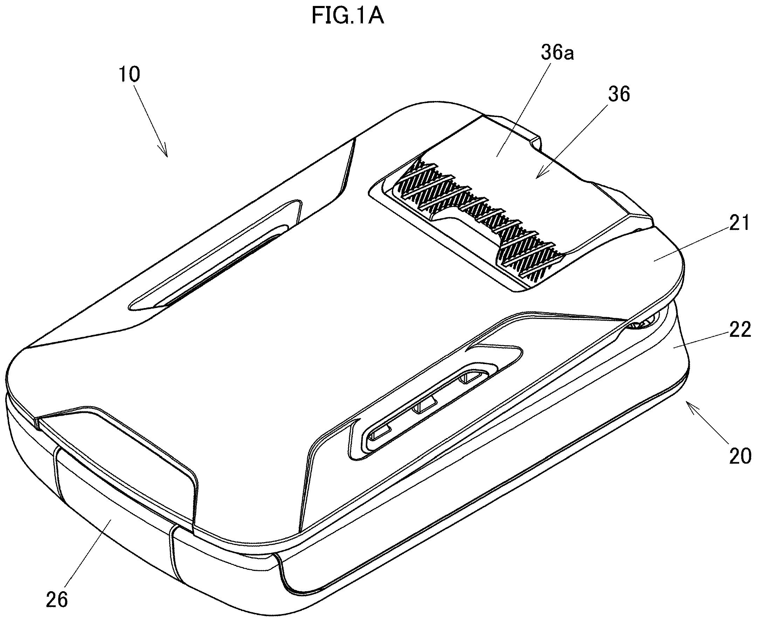

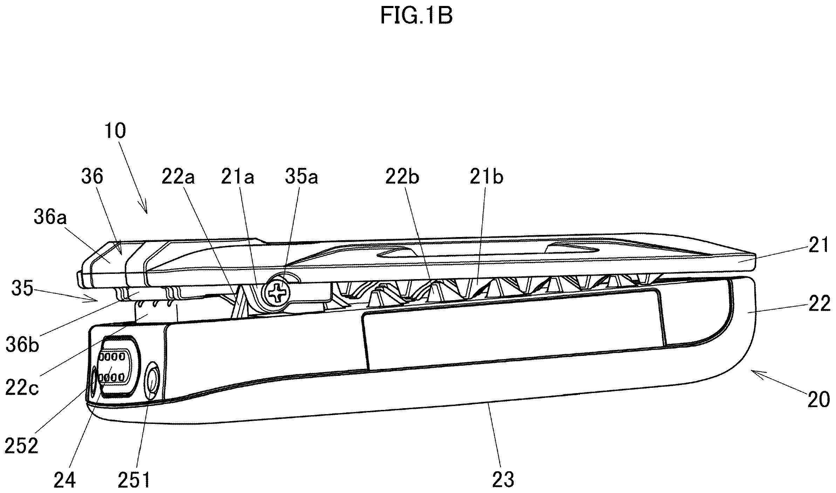

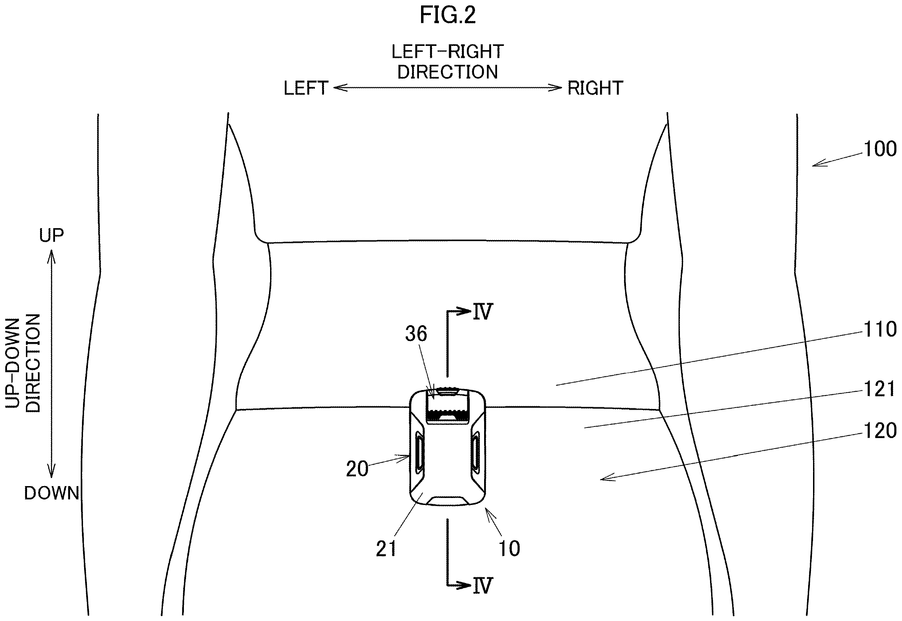

Referring to the accompanying drawings, an embodiment of the present disclosure will be described. A sensor device 10 shown in FIGs. 1A, 1B includes a main body portion 20, which is provided into a clip-like configuration by rotatably coupling a first main body portion 21 and a second main body portion 22 together. The main body portion 20 includes the first main body portion 21 and the second main body portion 22. The sensor device 10 is mounted on a body of a user 100 by holding an edge portion 121 (a rubber portion at the waist, a holding member) of clothing 120 (training pants in FIG. 2) worn by the user 100 using the clip-like main body portion 20, as shown in FIG. 2. Here, the edge portion 121, which constitutes the holding member, is such as to be worn by the user 100 at a certain part of the body of the user 100, and in the present embodiment, the edge portion 121 is worn by the user 100 at a waist part of the user 100. Then, the first main body portion 121 is disposed on an outer side of the clothing 120 (an outer side of the holding member which is an opposite side to a side facing the certain part of the body or an opposite side to a side facing the body of the user 100), and the second main body portion 22 is disposed on an inner side of the closing 120 (an inner side which is the side of the holding member which faces the certain part of the body or the side which faces the body of the user 100). A contact surface 23 (refer to FIG. 1B) is provided on the second main body portion 22. The contact surface 23 is provided on a surface of the second main body portion 22.

The sensor device 10 can be used by bringing the contact surface 23 into contact with a skin 110 on a back side of the user 100. When the user 100 takes exercise such as running, a movement of the body of the user 100, who is running, is detected and transmitted by a measuring unit 512 and a communication unit 524, respectively, which are possessed by the sensor device 10, so that the movement so detected and transmitted is analyzed at a terminal device such as a smartphone. The measuring unit 512 and the communication unit 524 will be described in detail later on.

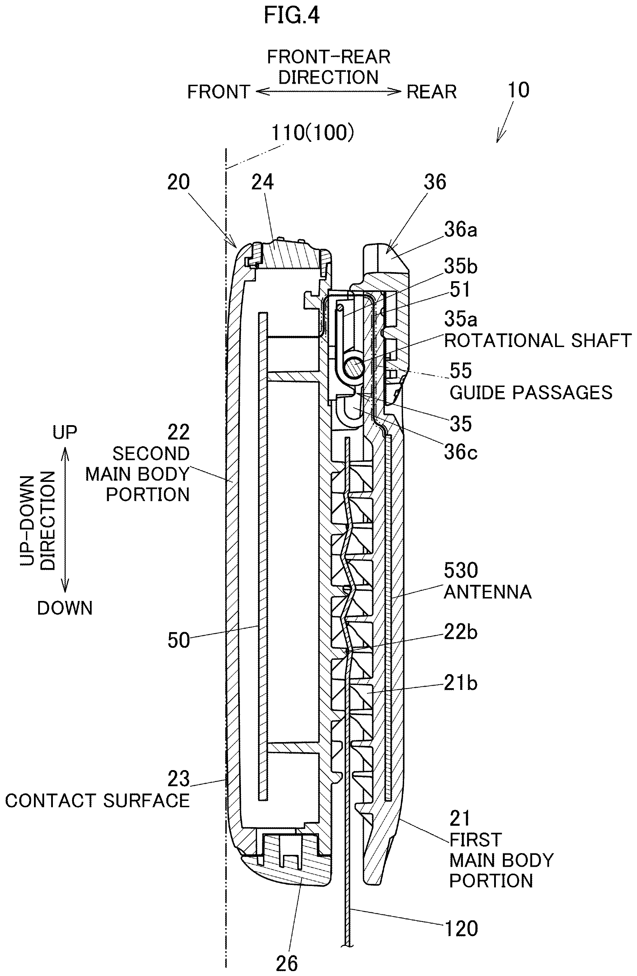

In the following description, when referring to an up-down direction, a left-right direction, and a front-rear direction of the sensor device 10, those directions are so referred to in such a state that the sensor device 10 is mounted on the body of the user 100 as described before. In the case of the up-down direction, an end of the sensor device 10 directed towards the head of the user 100 is referred to up, while an end of the sensor device 10 directed towards the legs of the user 100 is referred to as down. In the case of the left-right direction, a lateral side of the sensor device 10 directed to the left arm of the user 100 is referred to as left, while a lateral side of the sensor device 10 directed towards the right arm of the user 100 is referred to as right. In the case of the front-rear direction, a side of the sensor device 10 which is to be in contact with the skin 110 of the user 100 is referred to as front, while a side of the sensor device 10 which is opposite to the front side is referred to as rear, as shown in FIG. 4. In addition, in the following description, clockwise and counterclockwise rotations are referred to simply as rotation.

As shown in FIGs. 1A, 1B, the sensor device 10 has a substantially rectangular parallelepiped box-like shape which is long in the up-down direction. In the main body portion 20, the first main body portion 21 is provided on a rear side, while the second main body portion 22 is provided on a front side. The first main body portion 21 and the second main body portion 22 each have a substantially flat plate-like shape with flat surfaces oriented in the front-rear direction and are coupled together at a hinge portion 35, which is provided at an upper side of the main body portion 20.

The hinge portion 35 has a rotational shaft 35a. The rotational shaft 35a is passed through a bearing hole of a first bearing 21a, which projects from an upper portion on a front surface of the first main body portion 21, and a bearing hole of a second bearing 22a, which projects from an upper portion on a rear surface of the second main body portion 22, so as to couple the first main body portion 21 and the second main body portion 22 together in a rotatable fashion.

A torsion coil spring35b (refer to FIG. 4) is wound around a substantially central portion, in the left-right direction, of the rotational shaft 35a. As a result, the first main body portion 21 and the second main body portion 22, which are detachably attached to the edge portion 121, are biased so that portions which lie lower than the rotational shaft 35a move towards each other, whereby the sensor device 10 can be fixedly held to the user 100 via the clothing 120 by holding the edge portion 121 of the clothing 120 with the relevant lower portions of the first main body portion 21 and the second main body portion 22.

Multiple projections 21b, 22b are provided on the front surface of the first main body portion 21 and the rear surface of the second main body portion 22, respectively, so that the projections 21b, 22b can bite into the closing 120 so as to hold the closing 120 therebetween when the clothing 120 is held by the first main body portion 21 and the second main body portion 22.

A lock component 36 is provided at an upper end portion of the first main body portion 21. The lock component 36 includes a projecting portion 36b, which is provided on a front surface of a flat plate-like main body portion 36a thereof. An engagement hole 36c (refer to FIG. 4), which has an elongated hole-like shape, is provided in each of left- and right-hand sides of the projecting portion 36b. The rotational shaft 35a is passed through the engagement hole 36c for engagement therewith.

On the other hand, a push-up projecting portion 22c is provided at an upper end portion on the rear surface of the second main body portion 22 which lies opposite to the lock component 36 on the first main body portion 21. The lock component 36 is provided on the first main body portion 21 in such a manner as to move in the engagement hole 36c, which is formed into the elongated hole, in a longitudinal direction (substantially in the up-down direction) of the engagement hole 36c. When the user 100 operates the lock component 36 in such a manner as to move downwards, the projecting portion 36b is brought into abutment with the push-up portion 22c and then rides on the push-up portion 22c. As a result, even though the first main body portion 21 is caused to move towards the second main body portion 22 at an upper side than the rotational shaft 35a (that is, the first main body portion 21 is caused to move away or open from the second main body portion 22 at a lower side than the rotational shaft 35a), since the projecting portion 36b is in abutment with the push-up portion 22c, the first main body portion 21 is restricted from moving towards the second main body portion 22. In this way, the rotation of the second main body portion 22 about the rotational shaft 35a relative to the first main body portion is locked. To release the locked rotation of the second main body 22, the lock component 36 is moved upwards to release the state in which the projecting portion 36b rides on the push-up portion 22c. When the second main body portion 22 is released from the rotation-locked state, the first main body portion 21 can rotate about the rotational shaft 35a.

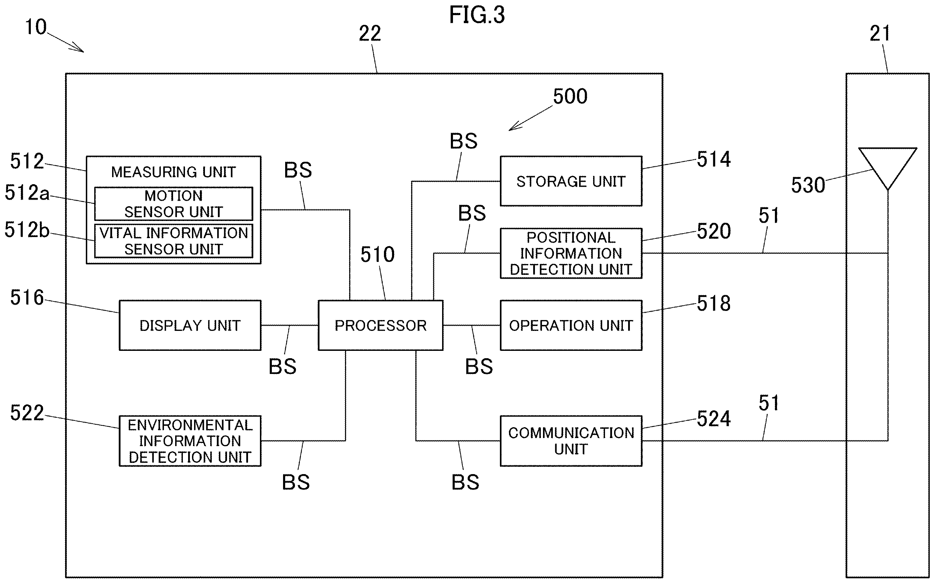

The second main body portion 22 has a substantially rectangular parallelepiped box-like shape which is long in the up-down direction and thick in the front-rear direction. The second main body portion 22 is formed as a case for accommodating a circuit board 50 (refer to FIG. 4) including a part of a circuit unit 500 (refer to FIG. 3), a battery (not shown) which constitutes a power supply, and the like. The front surface of the second main body portion 22 constitutes the contact surface 23 which can be brought into contact with the skin 110 of the user 100, while a rear surface of the second main body portion 22 faces a front surface of the first main body portion 21 with a slight gap defined therebetween. The contact surface 23 is described as being a flat surface but may be a curved surface.

A button switch 24, which functions as an operation unit 518, and two LEDs 251, 252, which function as a display unit 516, are provided on an upper surface of the second main body portion 22. When the user 100 operates and pushes down the button switch 24, operations can be performed such as supplying power to the sensor device 10, causing a measuring unit 512 to start detection, and the like, and a status of the sensor device 10 such as an operation mode or the like is indicated by illumination states of the LEDs 251, 252. A lid portion 26 is provided on a lower surface of the second main body portion 22 so as to cover a USB terminal (not shown).

Next, referring to FIG. 3, a control circuit of the sensor device 10 will be described. The circuit unit 500, which is provided in the second main body portion 22 of the main body portion 20 of the sensor device 10, includes a processor 510, the measuring unit 512, the display unit 516, the operation unit 518, a storage unit 514, a positional information detection unit 520, an environmental information detection unit 522, and the communication unit 524. These units are connected to the processor 510 by way of buses BS.

On the other hand, an antenna 530, which is configured to receive and transmit radio waves, is provided in the first main body portion 21 of the sensor device 10 (refer to FIG. 4). The antenna 530 is electrically connected to the circuit board 50, which includes the part of the circuit unit 500, by way of signal lines 51. Specifically, the antenna 530 is connected to the processor 510 by way of the communication unit 524 and is connected to the positional information detection unit 520 by way of the signal line 51, so that the antenna 530 can output a reception signal received from an outside device to the processor 510 by way of the positional information detection unit 520 and the communication unit 524. In addition, the antenna 530 can output a transmission signal to the outside device by way of the positional information detection unit 520 and the communication unit 524, which are controlled by the processor 510. In this way, the antenna 530 can communicate with the outside device. The outside device includes a terminal device such as a smartphone and a positioning satellite adopting the Global Navigation Satellite System (GNSS).

The processor 510 is made up of a Central Processing unit (CPU), which functions as at least one processor, a microcomputer, or the like and executes various types of processing by reading out programs stored in the storage unit 514. The storage unit 514 is made up of a flash memory, which functions as at least one memory, an Electrically Erasable Programmable ROM (EEPROM), and the like.

The storage unit 514 stores system programs and application programs that the processor 510 executes, as well as data necessary for the processor 510 to execute these programs. The measuring unit 512 measures information on the user 100. The measuring unit 512 has a motion sensor unit 512a and a vital information sensor unit 512b. The motion sensor unit 512a includes sensor elements such as a three-axis acceleration sensor, a gyro-sensor, a terrestrial magnetic sensor, and the like and can detect a movement of the subject unit (the sensor device 10) as a movement of a waist part of the user 100 who wears the sensor device 10, so that the motion sensor unit 512a outputs a detection signal so detected to the processor 510. The vital information sensor unit 512b includes various types of sensors for obtaining pulses of the user 100 and outputs a detection signal to the processor 510.

The display unit 516 includes the LEDs 251, 252, which are provided in, for example, the main body portion 20. The operation unit 518 includes, for example, the button switch 24 and outputs an operation signal, which indicates an operation state of the button switch 24 by the user 100, to the processor 510. Illumination states of the LEDs 251, 252 are controlled by the processor 510 in accordance with the operation signal indicating the operation state of the button switch 24 by the user 100.

The positional information detection unit 20 includes a Large Scale Integration (LSI) circuit having CPU, a memory, a filter, a modulator/demodulator, and the like and can derive positional information data of the sensor device 10 based on a reception signal from the positioning satellite received by way of the antenna 530, so that the positional information data so derived is output to the processor 510.

The environmental information detection unit 522 includes, for example, an atmospheric pressure sensor for measuring an atmospheric pressure at a place where the sensor device 10 (the main body portion 20) is located, and a detection signal detected by the environmental information detection unit 522 is output to the processor 510.

The communication unit 524 includes a circuit for transmitting and receiving a signal to and from the outside device (the terminal device) by way of the antenna 530 in a predetermined radio or wireless system, and this circuit has a filter, an amplifier, and a modulator/demodulator. The predetermined radio or wireless system is, for example, Bluetooth (a registered trademark), Bluetooth Law Energy (BLE) from Version 4 on of Bluetooth (the registered trademark), or the like. The communication unit 524 processes a transmission signal which is input from the processor 510, amplifies the transmission signal so processed, modulates the transmission signal so amplified, and transmits it to the outside device (the terminal device) by way of the antenna 530. In addition, the communication unit 524 demodulates a reception signal received by way of the antenna 530, amplifies the reception signal so demodulated, processes the reception signal so amplified, and outputs it to the processor 510.

The processor 510 executes various processing based on the detection signal detected by the measuring unit 512 (the motion sensor unit 512a, the vital information sensor module 512b), the positional information data obtained by the positional information detection unit 520, the detection signal detected by the environmental information detection unit 522 to thereby derive indexed data representing moving states of the user 100 such as a speed, a pitch, a stride, a vertical motion of the waist of the user 100 who is running or walking, and transmits the indexed data so derived as transmission signals to the outside device (the terminal device) by way of the communication unit 524. The outside device (the terminal device), for example, analyzes the movement of the user 100 based on the indexed data so transmitted.

In addition, as shown in FIG. 4, the main body portion 20 includes guide passages 55, which are formed of a through hole or a groove portion in the first main body portion 21 and the second main body portion 22, and the signal lines 51 are guided through the guide passages 55. The signal lines 55 electrically connect the circuit board 50, which includes the part of the circuit unit 500, and the antenna 530 together. The antenna 530 may include separately an antenna connected with the positional information detection unit 520 and an antenna connected with the communication unit 524.

When the sensor device 10 is attached to the closing 120, the first main body portion 21 is disposed on the outer side of the closing 120 (the edge portion 121), while the second main body portion 22 is disposed on the inner side of the closing 120. That is, the antenna 530 is disposed on the outer side of the closing 120. As a result, even if the closing 120 gets wet due to sweat exuded from the skin 110 of the user 100 as a result of movement or exercise, or rain, the antenna 530 is prevented from being held by the body of the user 100 which gets wet due to the sweat and the wet clothing 120 therebetween or is prevented from being covered with the wet closing 120, whereby the sensitivity of the antenna 530 is maintained in a good condition.

Thus, while the embodiment of the present disclosure has been described heretofore, the present disclosure is never limited by the embodiment so described, and hence, the present disclosure can be carried out while being modified variously. For example, while the circuit unit 500 is described as being provided in the second main body portion 22, in an example not according to the invention, a configuration may be adopted in which a second circuit unit including the measuring unit 512 (the motion sensor unit 512a) and the processor 510 is provided in the second main body portion 22, while the positional information detection unit 520 and the communication unit 524 (or one part of elements of the positional information detection unit 520 and one part of elements of the communication unit 524), as well as the antenna 530 are provided in the first main body portion 21. In the case that the one part of the elements of the positional information detection unit 520 and the one part of the elements of the communication unit 524 are provided in the first main body portion 21, the other parts of the positional information detection unit 520 and the communication unit 524 are provided in a first circuit unit.

The holding member is not limited to the edge portion 121 of the closing 120, and hence, the holding member can be a belt or the like. In addition, the sensor device 10 can be attached to a certain part of the body of the user 100 other than the back of the body. A clip-like attaching portion is adopted in the present embodiment.

Thus, according to the embodiments of the present disclosure, the sensor device 10 has the first main body portion 21 and the second main body portion 22, which are configured to be detachably attached to the edge portion 121 of the closing 120 which constitutes the holding member worn on a certain part of the body of the user 100. The first main body portion 21 is disposed on the outer side of the edge portion 121 which lies opposite to the side facing the waist part constituting the certain part of the body, and the antenna 530 for receiving or transmitting a radio wave is provided in the first main body portion 21. The second main body portion 22 is disposed on the inner side of the edge portion 121 which is the side facing the waist part constituting the certain part of the body, and the circuit unit 500 including the measuring unit 512 for measuring the information on the user 100 is provided in the second main body portion. As a result, the sensitivity of the antenna 530 can be improved irrespective of the states of the clothing 120 (whether the closing 120 is in the wet state due to sweat or in the dry state) .

The first main body portion 21 and the second main body portion 22 constitute the clip-like configuration by being coupled together rotatably. As a result, the sensor device 10 can easily be attached to the holding member. In addition, the circuit unit 500 further includes the processor 510, the positional information detection unit 520, and the communication unit 524. As a result, the sensor device 10 can easily be controlled. The measuring unit 512 includes the vital information sensor unit 512b for obtaining pulses and the motion sensor unit 512a for measuring the movement of the subject device. As a result, the motion and the status of the body when the high-degree movement is made can be analyzed. In addition, the second main body portion 22 includes the circuit board 50 which includes at least the part of the circuit unit 500. As a result, the individual units of the circuit unit 500 are accommodated in the second main body portion 22, and the measuring unit 512 for measuring the information on the user 100 is disposed close to at least the body, thereby making it possible to detect the information of the user with good precision.

In an example not according to the invention, the circuit unit 500 has the first circuit unit which is provided in the first main body portion 21 and which includes the positional information detection unit 520 and the communication unit 524 and the second circuit unit which is provided in the second main body portion 22 of the main body portion 20 which is disposed on the inner side of the clothing 120 and which includes the measuring unit 512 and the processor 510. As a result, the second main body portion 22, which is disposed close to the body, can be formed thin.

The first main body portion 21 and the second main body portion 22 have the guide passages 55 for guiding the signal lines 51 which electrically connect the antenna 530 with the circuit board 50. As a result, the signal lines 51 can be protected, while allowing the antenna 530 and the circuit board 50 to be connected electrically together in a proper fashion.

The second main body portion 22 includes the contact surface 23, which is configured to be in contact with the body of the user 100, on the surface which lies opposite to the surface facing the first main body portion 21. As a result, the second main body portion 22 including the measuring unit 512 is allowed to be brought into contact with the body on the contact surface 23, thereby making it possible to increase the measuring accuracy of the information on the user 100.

In addition, the circuit unit 500 includes the environmental information detection unit 522 for detecting an environment around the main body portion 20. As a result, a movement environment at the time of data collection can be recorded.

While the present disclosure has been described with reference to the exemplary embodiments, it is to be understood that the present disclosure is not limited to the disclosed exemplary embodiments. The scope of the following claims is to be accorded the broadest interpretation so as to encompass all such modifications and equivalent structures and functions.

## Claims

1. A sensor device (10) comprising:
a first main body portion (21) and a second main body portion (22) which are configured to be detachably attached to a holding member worn by a user (100), wherein the first main body portion (21) and the second main body portion (22) are provided into a clip-like configuration by being coupled together rotatably
an antenna (530) which is configured to receive radio waves,
a circuit unit (500) including a measuring unit (512) configured to measure information of the user (100) and a positional information detection unit which is configured to derive positional information data of the sensor device (10) based on a reception signal from a positioning satellite received by way of the antenna,
a circuit board (50) which includes at least a part of the circuit unit (500),
wherein the first main body portion (21) is configured to be disposed on a side of the holding member which lies opposite to a side facing a body of the user (100) and to accommodate therein the antenna (530), while the second main body portion (22) is configured to be disposed on the side of the holding member which lies to face the body of the user (100) and to accommodate therein the circuit board (50).

2. The sensor device (10) according to claim 1,
wherein the circuit unit (500) further comprises a processor (510) and a communication unit (524).

3. The sensor device (10) according to claim 1 or 2,
wherein the measuring unit (512) comprises a vital information sensor unit (512b) configured to obtain a pulse of the user and a motion sensor unit (512a) configured to measure a movement of a subject device.

4. The sensor device (10) according to any one of claims 1 to 3,
wherein the circuit unit (500) comprises a signal line which connects the antenna (530) with the circuit board (50), and the first main body portion (21) and the second main body portion (22) have a guide passage (55) configured to guide the signal line (51).

5. The sensor device (10) according to any one of claims 1 to 4,
wherein the second main body portion (22) comprises a contact surface (23) provided on an opposite side to a side facing the first main body portion (21) and configured to be brought into contact with the body.

6. The sensor device (10) according to any one of claims 1 to 5,
wherein the circuit unit (500) comprises an environmental information detection unit (522) configured to detect an environment around the sensor device (10).

## Patentansprüche

1. Eine Sensorvorrichtung (10), umfassend:
einen ersten Hauptkörperabschnitt (21) und einen zweiten Hauptkörperabschnitt (22), die so konfiguriert sind, dass sie lösbar an einem von einem Benutzer (100) getragenen Halteelement angebracht werden, wobei der erste Hauptkörperabschnitt (21) und der zweite Hauptkörperabschnitt (22) in eine clipartige Konfiguration vorgesehen sind, indem sie drehbar zusammen gekoppelt sind,
eine Antenne (530), die so konfiguriert ist, dass sie Radiowellen empfängt,
eine Schaltungseinheit (500), die eine Messeinheit (512), die so konfiguriert ist, dass sie Informationen des Benutzers (100) misst, und eine Positionsinformationsdetektionseinheit enthält, die so konfiguriert ist, dass sie Positionsinformationsdaten der Sensorvorrichtung (10) auf der Grundlage eines über die Antenne empfangenen Empfangssignals von einem Positionierungssatelliten ableitet,
eine Leiterplatte (50), die zumindest einen Teil der Schaltungseinheit (500) enthält,
wobei der erste Hauptkörperabschnitt (21) so konfiguriert ist, dass er auf einer Seite des Halteelements angeordnet ist, die einer einem Körper des Benutzers (100) zugewandten Seite gegenüberliegt, und dass er darin die Antenne (530) aufnimmt, während der zweite Hauptkörperabschnitt (22) so konfiguriert ist, dass er auf der Seite des Halteelements angeordnet ist, die dem Körper des Benutzers (100) zugewandt liegt, und dass er darin die Leiterplatte (50) aufnimmt.

2. Die Sensorvorrichtung (10) nach Anspruch 1,
wobei die Schaltungseinheit (500) weiterhin einen Prozessor (510) und eine Kommunikationseinheit (524) umfasst.

3. Die Sensorvorrichtung (10) nach Anspruch 1 oder 2,
wobei die Messeinheit (512) eine Vitalinformationssensoreinheit (512b) umfasst, die so konfiguriert ist, dass sie einen Puls des Benutzers erhält, und eine Bewegungssensoreinheit (512a), die so konfiguriert ist, dass sie eine Bewegung einer Subjektvorrichtung misst.

4. Die Sensorvorrichtung (10) nach irgendeinem der Ansprüche 1 bis 3,
wobei die Schaltungseinheit (500) eine Signalleitung umfasst, die die Antenne (530) mit der Leiterplatte (50) verbindet, und der erste Hauptkörperabschnitt (21) und der zweite Hauptkörperabschnitt (22) einen Führungsdurchgang (55) aufweisen, der konfiguriert ist, die Signalleitung (51) zu führen.

5. Die Sensorvorrichtung (10) nach irgendeinem der Ansprüche 1 bis 4,
wobei der zweite Hauptkörperabschnitt (22) eine Kontaktfläche (23) umfasst, die auf einer Seite vorgesehen ist, die einer dem ersten Hauptkörperabschnitt (21) zugewandten Seite gegenüberliegt, und die so konfiguriert ist, dass sie mit dem Körper in Kontakt gebracht wird.

6. Die Sensorvorrichtung (10) nach irgendeinem der Ansprüche 1 bis 5,
wobei die Schaltungseinheit (500) eine Umgebungsinformationsdetektionseinheit (522) umfasst, die so konfiguriert ist, dass sie eine Umgebung um die Sensorvorrichtung (10) herum detektiert.

## Revendications

1. Dispositif de capteur (10) comprenant :
une première section de corps principal (21) et une seconde section de corps principal (22) qui sont configurées pour être liées de façon amovible à un élément de support qui est porté par un utilisateur (100), dans lequel la première section de corps principal (21) et la seconde section de corps principal (22) sont constituées selon une configuration du type dispositif de serrage en étant couplées ensemble à rotation ;
une antenne (530) qui est configurée pour recevoir des ondes radio ;
une unité de circuit (500) qui inclut une unité de mesure (512) configurée pour mesurer une information de l'utilisateur (100) et une unité de détection d'information positionnelle qui est configurée pour dériver des données d'information positionnelle du dispositif de capteur (10) sur la base d'un signal de réception qui provient d'un satellite de positionnement et qui est reçu au moyen de l'antenne ; et
une carte de circuit (50) qui inclut au moins une partie de l'unité de circuit (500),
dans lequel la première section de corps principal (21) est configurée pour être disposée sur un côté de l'élément de support, lequel côté est situé à l'opposé d'un côté qui fait face au corps de l'utilisateur (100), et pour loger en son sein l'antenne (530), tandis que la seconde section de corps principal (22) est configurée pour être disposée sur le côté de l'élément de support qui est situé de manière à faire face au corps de l'utilisateur (100) et pour loger en son sein la carte de circuit (50).

2. Dispositif de capteur (10) selon la revendication 1,
dans lequel l'unité de circuit (500) comprend en outre un processeur (510) et une unité de communication (524).

3. Dispositif de capteur (10) selon la revendication 1 ou 2,
dans lequel l'unité de mesure (512) comprend une unité de capteur d'information vitale (512b) configurée pour obtenir un pouls de l'utilisateur et une unité de capteur de mouvement (512a) configurée pour mesurer un mouvement d'un dispositif sujet.

4. Dispositif de capteur (10) selon l'une quelconque des revendications 1 à 3,
dans lequel l'unité de circuit (500) comprend une ligne de signal qui connecte l'antenne (530) avec la carte de circuit (50), et la première section de corps principal (21) et la seconde section de corps principal (22) comportent un passage de guidage (55) configuré pour guider la ligne de signal (51).

5. Dispositif de capteur (10) selon l'une quelconque des revendications 1 à 4,
dans lequel la seconde section de corps principal (22) comprend une surface de contact (23) qui est prévue sur un côté opposé à un côté qui fait face à la première section de corps principal (21) et qui est configurée pour être amenée en contact avec le corps.

6. Dispositif de capteur (10) selon l'une quelconque des revendications 1 à 5,
dans lequel l'unité de circuit (500) comprend une unité de détection d'information environnementale (522) configurée pour détecter un environnement autour du dispositif de capteur (10).
